# EUROPEAN PATENT SPECIFICATION

(11) **EP 1 951 233 B1**
(45) Date of publication and mention of the grant of the patent: **28.03.2012**
(21) Application number: 06836744.0
(22) Date of filing: 30.10.2006
(51) Int. Cl.: A61K 31/4412, A61K 31/4745, A61K 31/522, A61K 31/519, A61K 31/7028, A61P 37/00

(54) **PIRFENIDONE/TOLL-LIKE RECEPTOR (TLR) AGONIST COMPOSITIONS AND METHODS FOR USING THEM TO STIMULATE PRODUCTION OF GRANULOCYTE COLONIZING STIMULATING FACTOR (G-CSF)**
ZUSAMMENSETZUNGEN VON PIRFENIDON/TOLL-LIKE REZEPTOR (TLR)-ANTAGONISTEN UND VERFAHREN ZU IHRER VERWENDUNG ZUR STIMULIERUNG DER PRODUKTION DES GRANULOZYTEN-KOLONIE-STIMULIERENDEN FAKTORS (G-CSF)
COMPOSITIONS DE PIRFENIDONE ET D'AGONISTES DU RECEPTEUR TOLL-LIKE ET LEURS METHODES D'UTILISATION POUR STIMULER LA PRODUCTION DU FACTEUR STIMULANT LA COLONISATION DES GRANULOCYTES

(30) Priority: 31.10.2005 US 731661 P
(43) Date of publication of application: 06.08.2008
(73) Proprietor: Intermune, Inc., Brisbane, CA 94005 (US)
(72) Inventor: PHILLIPS, Roderick, San Francisco, California 94117 (US); BLATT, Larry, San Francisco, California 94121 (US)
(74) Representative: Pilkington, Stephanie Joan
(86) International application number: PCT/US2006/042590
(87) International publication number: WO 2007/053658

(56) References cited:
- "Entry No 90236" PROUS INTEGRITY, 2005, XP002422444 Retrieved from the Internet: URL:www.prous.com> [retrieved on 2007-02-28]
- "Entry No 159623" PROUS INTEGRITY, 2005, XP002422445 Retrieved from the Internet: URL:www.prous.com> [retrieved on 2007-02-23]

## Description

### PRIORITY APPLICATIONS

This application claims priority to U.S. Provisional Application No. 60/731,661, filed October 31, 2005 .

### BACKGROUND OF THE INVENTION

### Field of the Invention

The present invention is directed to compositions and the use of compositions for treating subjects suffering from or at risk of developing neutropenia. In some embodiments, compositions comprising pirfenidone and one or more toll-like receptor (TLR) agonists are useful for stimulating production of granulocyte colonizing stimulating factor (G-CSF). In other embodiments, the compositions are for administrations to a subject suffering from or at risk of developing neutropenia, effective amounts of pirfenidone and one or more toll-like receptor (TLR) agonists.

### Description of the Related Art

Neutropenia is a haematological disorder characterized by an abnormally low number of a particular type of white blood cell, called a neutrophil. White blood cells, or leukocytes, circulate through the blood and are the main infection and disease-fighting cell of the human immune system. Neutrophils make up 50-70% of circulating white blood cells and serve as the primary defense against infections by destroying bacteria in the blood. Because the diminished number of neutrophils circulating in the blood substantially impairs the body's disease-fighting ability, patients suffering from neutropenia are at substantial risk of injection and disease. Without prompt medical attention, the condition may become life-threatening.

Neutropenia is typically discovered once a patient has developed severe infections or sepsis. Septecemia is an acute and overwhelming bacterial infection, wherein microbial antigens, such as lipopolysaccharides (LPS), initiate an uncontrolled release of host-derived pro-inflammatory mediators, which ultimately cause multi-organ failure and death. The survival rate of septecemia is less than 50% and tens of thousands (up to 200,000) die annually from septecemia-related incidents.

Common symptoms of neutropenia include fever, frequent infections, mouth ulcers, diarrhea, burning sensation when urinating, unusual redness, pain and/or swelling around a wound, sore throat, shortness of breath, and/or shaking chills.

Neutropenia is most commonly detected using a complete blood count (CBC) and is classified into four classes based on the absolute neutrophil count (ANC) of the patient. Measured in cells/mL of blood they are: (1) Neutropenia (AND=C) < 2000), (2) Mild Neutropenia (ANC between 1000 and 15000), (3) Moderate Neutropenia (ANC between 500 and 1000), and (4) Severe Neutropenia (ANC < 500). Neutropenia can also be detected and/or confirmed by bone marrow biopsy. Neutropenia lasting longer than 3 months is referred to as chronic neutropenia.

Severe, chronic neutropenia may be present at birth or may occur at any stage in life and is characterized by a selective decrease in the number of circulating neutrophils and an enhanced susceptibility to bacterial infections. There are several main types of severe, chronic neutropenia. Congenital neutropenia is a rare inherited form of the disease that affects children and may result in premature loss of teeth and/or peremptory gum infections. The most severe form of chronic congenital neutropenia is known as Kostmann's syndrome. Cyclic neutropenia is the rarest form of neutropenia. It typically occurs every three weeks, lasting six days at a time due to changing rates of cell production by the bone marrow. Idiopathic neutropenia is a rare form of neutropenia that develops in children and adults usually in response to an illness. It is diagnosed when the disorder cannot be attributed to any other disease and often causes life-threatening infections. Autoimmune neutropenia is most common in infants and young children and results when the body identifies neutrophils as non-self and produces antibodies to destroy them. Drug-induced neutropenia results following the use of certain drugs, toxins, radiation, chemotherapy and conventional oncology therapy. Many cancers have been found to be sensitive to extremely high doses of radiation or anti-neoplastic (anti-cancer) drugs. These cancers include malignant melanoma, carcinomas of the stomach, ovary, and breast, small cell carcinoma of the lung, and malignant tumors of childhood (including retinoblastoma and testicular carcinoma), as well as certain brain tumors, particularly glioblastoma. However, such intensive therapy is not always used because it frequently causes such a compromise of the hematopoietic system that the result is death due to any of numerous opportunistic infections.

Prous Science integrity database (www.prous.com) entry number 90236 identifies the chemical structure of Pirfenidone (5-Methyl-1-phenylpyridin-2(1H)-one) and provides a summary of its possible actions, and indications for which it has been trialled for therapy in humans.

Prous Science Integrity database (www.prous.com) entry number 159623 identifies the chemical structure of Loxoribine (7-Alkyl-B-oxo-7,8-dihydroguanosine) and provides a brief indication of its actions.

Current treatments for neutropenia include parenteral administration of recombinant granulocyte colony stimulating factor (G-CSF), bone marrow transplant, white cell transfusion, administration of cytokines, antibiotics, vitamins, and/or corticosteroids, and the like. There remains a need for an effective and convenient means to treat neutropenia, either by preventing or significantly reducing or eliminating the duration of neutropenia for parents suffering from severe chronic neutropenia (congenital, idiopathic, cyclic, autoimmune, or drug-induced). In addition, there is a need for a treatment that could be used to treat prevent neutropenia in a patient who is at risk for developing neutropenia, or who, although not suffering from the disease, has a reduced level of neutrophils

### SUMMARY OF THE INVENTION

Disclosed herein are novel compositions and medicaments for use in treating and/or inhibiting neutropenia in a subject in need thereof. In preferred embodiments, the compositions comprise a therapeutically effective amount of pirfenidone co-formulated with one or more toll-like receptor agonists.

In some embodiments of the invention disclosed herein, the compositions and medicaments are for administration of a therapeutically effective amount of pirfenidone and one or more toll-like receptor (TLR) agonits to said subjects.

In some embodiments, the compositions and medicaments are for administration of pirfenidone and said TLR agonists in an amount effective for increasing the number of neutrophils in the subject. In some embodiments, the therapeutically effective amount is less than 50% of an amount that causes an undesirable side effect in the subject.

In some embodiments, the compositions and medicaments are for administration of said pirfenidone and said one or more TLR agonists simultaneously. In preferred embodiments, the pirfenidone and one or more TLR agonists are co-formulated and are for administration in combination with a pharmaceutically acceptable carrier. Preferably, the compounds disclosed herein are for oral administration. Thus, in some embodiments, the compositions and medicaments are for administration of a tablet or capsule, wherein the tablet or capsule comprises said pirfenidone and one or more TLR agonists.

In some embodiments, one or more tablets or capsules are for administration to the subject one or more times per day. In some embodiments, one or more of capsules are for administration to the subject twice per day. In other embodiments, one or more capsules are for administration to the subject three times per day.

In some embodiments, the composition a medicament is for providing the pirfenidone in a dose of from about 100 to about 400 milligrams. In some embodiments, the compositions and medicaments are for administration of the pirfenidone such that the daily intake is from about 800 to about 4000 mg/day. In some embodiments, the compositions and medicaments are for administration of the pirfenidone such that the daily intake is about 1200 mg/day or higher.

In some embodiments, the neutropenia is severe neutropenia. The neutropenia may be selected from the group consisting of neutropenia associated with chemotherapy, neutropenia associated with conventional oncology therapy, drug-induced neutropenia, disease-induced neutropenia, genetic neutropenia, toxin-induced neutropenia, and radiation-induced neutropenia. In some embodiments, the neutropenia is congenital neutropenia. In other embodiments, the neutropenia is cyclic neutropenia. In still other embodiments, the neutropenia is idopathic neutropenia.

In a preferred embodiment, the one or more TLR agonists comprises at least one TLR7 agonist. The TLR 7 agonist may be selected from the group consisting of 7-thia-8-oxoguanosine, 7-deazaguanosine, 7-allyl-8-oxoguanosine, 7-dezaguanosine, imiquimod, and R848.

### BRIEF DESCRIPTION OF THE DRAWINGS

Figure 1 is a line graph showing the effects of pirfenidone (185 µg/mL) and LPS (1µg/mL) on TNF- α release in PBMCs.

Figure 2 is a line graph showing the effects of pirfenidone (inM) and LPS (1 µg/mL) on TNF-α release in PBMCs.

Figure 3 is a line graph showing the effects of LPS and pirfenidone at varying concentrations (0-5,000µM) on TNF-α release at 8 hours in PBMCs.

Figure 4 is a line graph showing the effects of LPS (1,000 ng/mL or 0.1 ng/mL) and pirfenidone at varying concentrations (0-5,000µM) on TNF-α release at 24 hours in PBMCs.

Figure 5 is a line graph shwoing the concentration dependence of pirfenidone effect in LPS-mediated induction of TNF-α.

Figure 6 is a line graph showing the effects of LPS (1 µg/ml ) and pirferidone (1 µM) on G-CSF release in PBMCs at 0, 2, 4, 8, and 24 hours.

Figure 7 is a line graph showing the effects of LPS' (1,000 ng/mL, 100 ng/mL, 10 ng/mL, or 1 ng/mL) and pirfenidone at varying concentrations (0-5,000µM) on G-CSF release in PBMCs at 8 hours.

Figure 8 is a line graph showing the effects of LPS (1 µg/ml or 0.1, ng/ml and varying concentrations of pirfenidone (0-5,000 µM) on G-CSF release in PMBCs at 24 hours.

Figure 9 is a bar graph showing the effects of pirfenidone (185 mg/mL) and LPS (1 mg/mL) on cytokine release in PBMCs.

Figure 10 is a bar graph showing that TNF-α release is inhibited for all TLR agonists.

Figure 11 is a bar graph showing that G-CSF release was augmented for all TLR agonists.

Figure 12 is a bar graph showing the effect of various p38 inhibitors on LPS-mediated TNF- a and G-CSF release from human PBMCs. The results are shown as fold induction.

### DETAILED DESCRIPTION OF THE PREFERRED EMBODIMENT

It has now been discovered that a high therapeutic effect in treating neutropenia may be achieved using pirfenidone in combination with one or more toll-like receptor (TLR) agonists.

Neutropenia is characterized by an abnormally low number of a particular type of white blood cell, called a neutrophil. Neutrophils form part of the innate immune response. They kill bacteria via phagocytosis and the respiratory burst. Granulocyte colonizing stimulating factor (G-CSF) is a hematopoietic growth factor for neutrophil development. Endogenous G-CSF is produced by monocytes, fibroblasts, and, endothelial cells. It is one of the surprising discoveries of the invention that pirfenidone in combination with one or more TLR agonists inhibits TNF-α release and augments G-CSF release. According, compositions and medicaments for use in treating subjects suffering from or at risk of developing neutropenia are disclosed herein. Embodiments of the invention provide compositions and medicaments for use in treating or preventing neutropenia that may result from chemotherapy, conventional oncology therapy, drugs, diseases, genetic disorders, toxins, and radiation, as well as compositions and medicaments for use in treating and/or preventing neutropenia in subjects exhibiting reduced populations of neutrophils.

In one embodiment, the invention provides for the use of effective amounts of pirfenidone in combination with one or more TLR agonists. Examples of TLR agonists useful in the invention are described herein and discussed more fully below. In a preferred embodiment, the TLR agonist is a TLR7 agonist.
The term "at risk for or suffering from" as used herein, refers to subjects having a high probability of acquiring or developing neutropenia and/or subjects suffering from neutropenia or a condition associated with neutropenia, and includes, for example, subjects currently experiencing neutropenia and those not currently experiencing neutropenia but undergoing a therapy known to cause neutropenia. The term also includes subjects who, although not suffering from severe neutropenia, have a reduced population of neutrophils. Methods for identifying a subject at risk for or suffering from neutropenia or a condition known to cause neutropenia are known in the art.

In some embodiments, the compositions and medicaments are for use in treating a patient currently experiencing neutropenia. In another embodiment, the compositions and medicaments are for use in treating a patient diagnosed with cyclic neutropenia but not currently experiencing a low neutrophils count. In still another embodiment, the compositions and medicaments are for use in treating a patient who has not been diagnosed with neutropenia, but who has been identified as being at risk for developing neutropenia. Risk factors of neutropenia are well known in the art, and include, but are not limited to, bacterial or viral infections, certain drug usage, and cancer therapies, such as myelosuppressive chemotherapy, induction or consolidation chemotherapy, bone marrow transplant, peripheral blood progenitor cell collection and therapy, and the like. Thus, in some embodiments, pirfenidone in combination with one or more TLR agonists may be for administration to a patient receiving a cancer therapy. Pirfenidone and a TLR agonist are for administration prior to, during, or after such therapies to prevent or treat neutropenia resulting from such therapies or to combat infection, thereby reducing, if not completely eliminating, the risks of morbidity and death.

Subjects exhibit or are at risk for developing neutropenia in several clinical situations. For example, subjects may exhibit neutropenia after bacterial or viral infection. Post-infectious neutropenia can start within a few days of the onset of an infection and last several weeks. Examples of viral and bacteria agents that give rise to neutropenia include varicella, measles, rubella, hepatitis A and B, infectious mononucleosis and influenza, human-immunodeficiency virus (HIV), brucellosis, tularemia, riekettsia, and M. tuberculosis.

In addition, subjects may exhibit drug-induced neutropenia following administration of anti-neoplastic agents or other drugs that suppress bone marrow. The neutropenia manifests in about one two weeks after exposure to these drugs. The degree of neutropenia depends upon the dose and duration of exposure. Recovery usually occurs within a few days of stopping the drug, however, marrow recovery may take 10 to 14 days. Often the medication is essential for the patient and therefore, may be continued under close monitoring, provided absolute neutrophil count is above 500 to 700 and there is no active infection. Such drugs include, for example, but are not limited to, Dipyrone, Mianserin, Sulfasalazine, Co-trimoxazole, Anti-arrythmic agents, Procainamide, Ajmaline, Tocainide, Aprindine, Amiodarone, Penicillins, Amoxycillin, Aziocillin, Benzylpenicillin, Phenethicillin, Cloaxacillin and penicillin, Thiouracil derivatives, Methyl thiouracil, Propyl thiourcil, Phenylbutazone, Cimetidine, Penicillamine, Diclofenac, Carbamazepine, ACE-Inhibitors, Captopril, Enalapril, Hydrochlorothiazide with potassium sparing diuretics, Indomethacine, Cephalosporins, Cephalexin, Cepahazolin, Cefuroxime, Cefitaxime, Cephradine, Oxyphenbutazone, Nitrofurantoin, Salicylic acid derivatives, Clozapine, Carbimazone, Sulphonylurea derivatives, Glibenclamide, Tolbutamide, Methyldopa, Thiamazole, Nucleosides, Aminoglutethimide, Ibuprofen, Pentazocine, Levamizole, Promethazine, Chloramphinicol, Acetaminophen and combinations, Perazine, Mebhydrolin, Ranitidine, and Imipramine.

Drugs with relatively lower but still significant probability of inducing neutropenia include, for example, Phenytoin, Chlorthalidone, Sulphamethizole, Norfloxacin, Naproxen, Clomipramine, Trazodone, Omeprazole, Alimemazine, Pirenzepine, Ticlopidine, Ibopamine, Hydralazine, Nifedipine, Nalidixic acid, Doxycycline, Clindamycin, Gentamycin, Fusidic acid, Dapsone, Azapropazone, Propyphenazone, Sulindac, Piroxicam, Pirprofen, Niflumic acid, Allopurinol, Glafenine, Valproate, Levadopa with carbidopa, Chlorpramazine, Haloperidol, spironolactone, Zuclopenthixol, Zopiclone, Cinnarizine, Metronidazole, Pyrimethamine combinations, and Thophylline.

Neutropenia may also be associated with immunologic abnormalities, (autoimmune neutropenia), metabolic diseases, hypersplenism, and nutritional deficiencies.

In preferred embodiments, the compositions and medicaments are for administration of a composition comprising pirfenidone and one or more TLR agonist as described below. Preferably, the route of administration is oral

Pirfenidone is small drug molecule whose chemical name is 5-methyl-1-phenyl-2-(1H)-pyridone. It is a non-peptide synthetic molecule with a molecular weight of 185.23 daltons. Its chemical elements are expressed as C12H11NO, and its structure and synthesis are known. Pirfenidone is manufactured commerically and being evaluated clinically as a broad-spectrum anti-fibrotic drug. Pirfenidone has anti-fibrotic properties via: decreased TNF-α expression, decreased PDGF expression, and decreased collagen expression. Several pirfenidone Investigational New Drug Applications (INDs) are currently on file with the U.S. Food and Drug Administration. Phase II human investigations are ongoing or have recently been completed for pulmonary fibrosis, renal glomerulosclerosis, and liver cirrhosis. There have been other Phase II studies that used pirfenidone to treat benign prostate hypertrophy, hypertrophic scarring (keloids), and rheumatoid arthritis.

"Toll-like receptor" (TLR), as used herein, refers to any of a family of type I transmetnbrane signaling receptor proteins that are homologous to the Drosophila melanogaster Toll protein. TLRs recognize a variety of microbial nucleic acid-derivatives, metabolites, and products to induce activation of NF-κB and other signaling pathways to activate the innate immune system. TLRs recognize a wide variety of ligands, called pathogen-associated molecular-patterns (PAMPs), discriminating gram-positive and gram-negative bacteria from fungi and other pathogens. Ten members of the TLR family have been so far identified in humans, and are called human TLR1 through human TLR10. Rock F L et al., PNAS 95:588-593 (1998); Chaudhary P M et al., Blood 91:4020-4027 (1998); Takeuchi O. et al., Gene 231:59-65 (1999); Aderem A. et al., Nature 406:782-7 (2000). Genetic data obtained to date indicate that the TLRs have unique functions and are not redundant.

TLR proteins comprise an extracellular domain containing leucine-rich repeats (LRRs) domains, a C-terminal flanking region (LRRCT), and an intercellular domain containing a cytoplasmic signaling domain, that is, a so-called Toll/interleukin-1 receptor homology domain (Toll/IL-1R domain: TIR domain). L. A. O' Neil and C. A. Dinarello, Immunol. Today 21 (2000) 206-209). A typical LRR has a repeat structure consisting of 24 amino acids containing conserved asparagine residual groups and leucine residual groups, and is included in various proteins of bacteria, yeasts, plants, and animals, so that LRR domain is considered to act upon protein-protein interaction.

A "TLR agonist" as used herein, refers to a substance that can combine with a TLR and activate it. By slightly altering the structure of such substances, TLR agonists can be designed to have different stabilities in the body, allowing a certain amount of control over where the substances go, and how long they last. Microbial ligands have been identified for several mammalian TLRs. For example, TLR4 recognizes lipopolysaccharide (LPS), TLR2 interacts with peptidoglycan, bacterial lipopeptides, and certain types of LPS, TLR3 recognizes double-stranded RNA, TLR5 recognizes bacterial flagellin, TLR9 recognizes bacterial DNA. Lee et al., PNAS 100:6646-51 (2003).

In some embodiments, the TLR agonist is a nucleoside, and preferably, a nucleoside that activates TLR7. TLR7 is involved in the response to viral infection and recognizes GU-rich short single-stranded RNA as well as small antiviral compounds and small synthetic molecules. TLR7 agonists include, for example, but are not limited to, guanosine analogs having substituents at the 7- and/or 8-positions, such as 7-thia-8-oxoguanosine (also referred to as or TOG or isatoribine), 7-deazaguanosine, 7-allyl-8-oxoguanosine (loxoribine), 7-dezaguanosine (7-deza-G), imiquimod (R837), and R848.

As used herein, an "effective amount of a TLR agonist" refers to an amount which when administered orally, subcutaneously, intramuscularly, intravenously, by aerosol to the respiratory tract, intradermally, or rectally, induces a biological response in the individual. Such response is manifested by a stabilization or improvement in immune system function and, in particular, neutrophil counts.

Subjects receiving pirfenidone in combination with one or more TLR agonists preferably exhibit fewer opportunistic infections and consequently demonstrate better response to chemotherapy or other therapy for infectious diseases. Treated subjects may require less hospitalization and exhibit an overall improvement in general clinical condition. When administered to subjects who experience autoimmune neutropenia, hypersplenism, some metabolic diseases and some nutritional deficiencies, the development of fatal infection with nonpalhogenic bacteria may be prevented. Subjects treated using the compositions and medicaments described herein preferably perceive an improvement in the quality of life. The preferred composition comprises pirfenidone and a TLR7 agonist, is well tolerated and may be administered with concurrent neutropenia therapies.

The compositions and medicaments disclosed herein use for administration of both pirfenidone and a TLR agonist to a subject suffering from or at risk of developing neutropenia. Preferably, pirfenidone and the one or more TLR agonists are for delivery to the same site and may be co-formulated, e.g. mixed together, co-administered, conjugated together, etc., or formulated separately, depending on the requirements of the specific agents. The TLR agonist(s) and pirfenidone can be for delivery simultaneously or within a short period of time, by the same or by different routes. In a preferred embodiment, the TLR agonist and pirfenidone are co-formulated, meaning that they are for delivery together as part of a single composition. Preferably, the resulting single composition is formulated such that the optimum ratio of pirfenidone to the one or more TLR agonists is achieved. The TLR agonist(s) and pirfenidone may be associated with one another by covalent linkage, or by non-covalent interaction such as hydrophobic interaction, hydrogen bonding, ionic interaction, van der Walls interaction, magnetic interaction, or combinations thereof. Alternatively, the TLR agonist(s) and pirfenidone may simply, be mixed in a common suspension. In addition, the TLR agonist(s) and pirfenidone may be encapsulated together in some form of delivery device such as, for example, an alginate device, a liposome, chitosan vesicle, etc.

As used herein, "in association with" refers to a reversible union between two chemical entities, whether alike or different, to form a more complex substance.

"In combination with" refers to either a reversible or irreversible (e.g. covalent) union between two chemical entities, whether alike or different, to form a more complex substance.

"Linker" refers to any chemical entity that links one chemical moiety to another chemical moiety. Thus, something that chemically or physically connect pirfenidone and one or more TLR agonists is a linker. Examples of linkers include, but are not limited to, complex or simple hydrocarbons, nucleosides, nucleotides, nucleotide phosphates, oligonucleotides, polynucleotides, nucleic acids, amino acids, small peptides, polypeptides, carbohydrates (e.g., monosaccharides, disaccharides, trisaccharides), and lipids. Without limitation, the present invention also contemplates using a peptide bond or an amino acid or a peptide linker to link pirfenidone and a toll-like receptor. The invention further contemplates preparing such a Linked molecule by recombinant DNA procedures. A linker can also function as a spacer.

"Spacer" refers to any chemical entity placed between two chemical moieties that serves to physically separate the latter two moieties. Thus, a chemical entity placed between pirfenidone and one or more TLR agonists is a spacer. Examples of spacers include, but are not limited to, nucleic acids (e.g. untranscribed DNA between two stretches of transcribed DNA), amino acids, carbohydrates (e.g., monosaccharides, disaccharides, trisaccharides), and lipids.

The dose and protocol for delivery of the TLR agonist will vary with the specific TLR agonist selected. Typically one or more doses are administered.

A preferred subject is a mammal. A mammal may include any mammal. As a non-limiting example, preferred mammals include cattle, pigs, sheep, goats, horses, camels, buffalo, cats, dogs, rats, mice, and humans. A highly preferred subject mammal is a human. The compound(s) may be for administration to the subject via any drug delivery route known in the art, including for example, but not limited to, oral, ocular, rectal, buccal, topical, nasal, ophthalmic, subcutaneous, intramuscular, intravenous (bolus and infusion), intracerebral, transdermal, and pulmonary. Preferably, the compounds are for administration to the subject orally.

The terms "therapeutically effective amount" and "prophylactically effective amount," as used herein, refer to an amount of a compound sufficient to treat, ameliorate, or prevent the identified disease or condition, or to exhibit a detectable therapeutic, prophylactic, or inhibitory effect. For example, the effect may be restoration of normal absolute neutrophil count (ANC), increased ANC, prevention of infection, febrile neutropenia, decreased hospitalization, decreased antibiotic usage, reduction of the incidence, severity, and duration of severe neutropenia, prevention of recurrence of neutropenia, prevention of developing neutropenia, and the like. The effect maybe detected by any means known in the art. The precise effective amount for a subject will depend upon the subject's body weight, size, and health; the nature and extent of the condition; and the therapeutic or combination of therapeutics selected for administration. Therapeutically and prophylactically effective amounts for a given situation may be determined by routine experimentation that is within the skill and judgment of the clinician.

For any compound, the therapeutically or prophylactically effective amount may be estimated initially either in cell culture assays or in animal models, usually rats, mice, rabbits, dogs, or pigs. The animal model may also be used to determine the appropriate concentration range and route of administration. Such information may then be used to determine useful doses and routes for administration in humans.

Therapeutic/prophylactic efficacy and toxicity may be determined by standard pharmaceutical procedures in cell cultures or experimental animals, e.g., ED₅₀ (the dose therapeutically effective in 50% of the population) and LD₅₀ (the dose lethal to 50% of the population). The dose ratio between therapeutic and toxic effects is the therapeutic index, and it may be expressed as the ratio, ED₅₀/LD₅₀. Pharmaceutical compositions that exhibit large therapeutic indices are preferred. However, the pharmaceutical compositions that exhibit narrow therapeutic indices are also within the scope of the embodiments. The data obtained from cell culture assays and animal studies may be used in formulating a range of dosage for human use. The dosage contained in such compositions is preferably within a range of circulating concentrations that include an ED₅₀ with little or no toxicity. The dosage may vary within this range depending upon the dosage form employed, sensitivity of the patient, and the route of administration.

More specifically, the maximum plasma concentrations (Cₘₐₓ) of pirfenidone and the TLR agonist(s) may range from about 65 µM to about 115 µM, or about 75 µM to about 105 µM, or about 85 µM to about 95 µM, or about 85 µM to about 90 µM depending upon the route of administration. In general the dose will be in the range of about 100 mg/day to about 10 g/day, or about 200 mg to about 5 g/day, or about 400 mg to about 3 g/day, or about 500 mg to about 2 g/day, in single, divided, or continuous doses for a patient weighing between about 40 to about 100 kg (which dose may be adjusted for patients above or below this weight range, particularly children under 40 kg). Generally the dose will be in the range of about 25 mg/kg to about 300 mg/kg of body weight per day. The dosing may be once, or twice or three times daily, with one or more units per intake. In one embodiment, the co-formulated compound is for administration to the subject in a unit dosage form comprising about 100 to about 400 mg of pirfenidone per dose.

In some embodiments, the pirfenidone and TTR agonist(s) are present in quantities that produce a mutual synergistic effect on the augmentation of G-CSF expression and/or in the treatment or prevention of neutropenia. The term "syriergistic" as used herein means that the effect achieved with the compounds used together is greater than the sum of the effects that result from using the compounds separately. In a preferred embodiment, the level of synergism is more than 10% of the effect that would be expected based on the rule of mixtures. In a more preferred embodiment, the level of synergism is about 20% or about 30% greater. In a more preferred embodiment, the level of synergism is about 40% or 50% greater.

The exact dosage will typically be determined by the practitioner, in light of factors related to the subject that requires treatment. Dosage and administration are generally adjusted to provide sufficient levels of the active agent(s) or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulations.

It will be appreciated that treatment as described herein includes preventing a disease, ameliorating symptoms, slowing disease progression, reversing damage, or curing a disease.

Treating neutropenia with the compositions and medicaments of the invention may result in an increase in absolute neutrophil count (ANC) of treated subjects relative to the ANC prior to treatment. Preferably, the average ANC is increased by more than about 2 fold; more preferably, by more than about 3 fold; more preferably, by more than about 5 fold; and even more preferably by more than about 7 fold. An increase in ANC may be measured by any reproducible means. An increase in average ANC may be measured, for example, by performing a complete blood count (CBC) following initiation of treatment with an active compound. An increase in average ANC of a population may also be measured, for example, by performing a CBC following completion of a first round of treatment with an active compound.

Treating neutropenia may result in a reduction in the incidence of infection. Preferably, the incidence of infection of a treated population is reduced by at least about 25% relative to the incidence of infection of an untreated population; more preferably, the incidence of infection is reduced by at least about 30%; more preferably, reduced by at least about 33%; more preferably, reduced by at least about 40%; more preferably, reduced by at least about 50%; more preferably, reduced by at least about 60%; even more preferably, reduced by at least 70%; and most preferably, reduced by at least about 75%. Incidence of infection may be measured by any reproducible means of measurement.

Treating neutropenia may result in a reduction of hospitalization. Preferably, in-patient hospitalization of treated patients is reduced by at least about 25% relative to the in-patient hospitalization of an untreated population; more preferably, by at least about 30%; more preferably, by at least about 40%; more preferably, by at least about 45%; more preferably, by at least about 50%; more preferably, by at least about 60%; even more preferably, by at least about 70%; and most preferably, by at least about 75%. In-patient hospitalization may be measured by any reproducible means of measurement.

Treating neutropenia may result in a reduction in antibiotic usage. Preferably, antibiotic usage of a treated population is reduced by at least 50% relative to an untreated population; more preferably, reduced by at least about 60%; more preferably, reduced by at least about 65%; more preferably, reduced by at least about 70%; more preferably, reduced by at least about 75%; more preferably, reduced by at least about 80%; even more preferably, reduced by at least about 85%; and most preferably, reduced by at least about 90%. Reduction in antibiotic usage may be measured by any reproducible means of measurement.

Treating neutropenia may result in an increase in average survival time of a population of treated subjects in comparison to a population of untreated subjects. Preferably, the average survival time is increased by more than about 30 days; more preferably, by more than about 60 days; more preferably, by more than about 90 days; and even more preferably by more than about 120 days. An increase in survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following complexion of a first round of treatment with an active compound.

Treating neutropenia may result in a decrease in the mortality rate of a population of treated subjects in comparison to a population of subjects receiving carrier alone. Treating neutropenia may result in a decrease in the mortality rate of a population of treated subjects in comparison to an untreated population. Treating neutropenia may result a decrease in the mortality rate of a population of treated subjects in comparison to a population receiving monotherapy with a drug that is not a compound of the embodiments, or a pharmaceutically acceptable salt, metabolite, analog or derivative thereof. Preferably, the mortality rate is decreased by more than about 2%; more preferably, by more than about 5%; more preferably, by more than about 10%; and most preferably, by more than about 25%. A decrease in the mortality rate of a population of treated subjects may be measured by any reproducible means. A decrease in the mortality rate of a population may be measured, for example, by calculating for a population the average number of disease-related deaths per unit time following initiation of treatment with an active compound. A decrease in the mortality rate of a population may also be measured, for example, by calculating for a population the average number of disease related deaths per unit time following completion of a first round of treatment with an active compound.

Treating neutropenia may result in an increase in G-CSF expression. Preferably, after treatment, G-CSF expression is increased by at least about 5%;
more preferably, by at least about 10%; more preferably, by at least about 20%; more preferably, by at least about 30%; more preferably, by at least about 40%; more preferably, by at least about 50%; even more preferably, by at least about 60%; and most preferably, by at least about 75%. Increase in G-CSF expression may be measured by any reproducible means of measurement.

As described elsewhere herein, the compounds described herein may be formulated in pharmaceutical compositions, if desired and may be for administration by any route that permits treatment of the disease or condition. A preferred route of administration is oral administration. Administration may take the form of single dose administration, or the compound of the embodiments may be for administration over a period of time, either in divided doses or in a continuous-release formulation or administration method (e.g., a pump). However the compounds of the embodiments are administered to the subject, the amounts of compound administered and the route of administration chosen should be selected to permit efficacious treatment of the disease condition.

The invention includes the use of pirfenidone together with one or more TLR agonists for the treatment of disease conditions. TLR agonists are well-known in the art and include, for example, but not limited to, lipopolysaccharide (LPS, binds TLR4), Fibrin (binds TLR4), lipoteichoic acid (LTA, binds TLR2), peptidoglycan (PG, binds TLR2), or CpG (bacterial DNA, binds TLR9), 7-thia-8-oxoguanosine (TOG or isatoribine, binds TLR7), 7-deazaguanosine (binds TLR7), 7-allyl-8-oxoguanosine (loxoribine, binds TLR7), 7-dezaguanosine (7-deza-G, binds TLR7), imiquimod (R837, binds TLR7), or R848 (binds TLR7). The combination of active ingredients may be: (1) co-formulated and administered or delivered simultaneously in a combined formulation; (2) delivered by alternation or in parallel as separate formulations; or (3) by any other combination therapy regimen known in the art. When they use for delivery in alternation therapy, the compositions and medicaments described herein may be for administration a delivery of the active ingredients sequentially, e.g., in separate solution, emulsion, suspension, tablets, pills or capsules, or by different injections in separate syringes. In general, during alternation therapy, an effective dosage of each active ingredient is administered sequentially, i.e., serially, whereas in simultaneous therapy, effective dosages of two or more active ingredients are administered together. Various sequences of intermittent combination therapy may also be used.

In addition, embodiments of the invention include the use of a compound or compounds as described herein together with one or more other neutropenia therapies. Neutropenia therapies are well-known in the art, and include, for example, treatment with racombinant granulocyte-colony stimulating factor (G-CSF), bone marrow transplant, white cell transfusion, administration of cytokines, antibiotics, vitamins, and/or corticosteroids, and the like. Thus, for example, the compounds described herein may be administered before, during or after one or more neutropenia therapies.

### Pharmaceutical Compositions

While it is possible for the compounds useful in the invention described herein to be administered alone, it may be preferable to formulate the compounds as pharmaceutical compositions. As such, the present invention provides pharmaceutical compositions according to the invention. More particularly, the pharmaceutical compositions described herein may be useful, *inter alia*, for treating or preventing neutropenia. A pharmaceutical composition is any composition that may be administered in vitro or *in vivo* or both to a subject in order to treat or ameliorate a condition. In a preferred embodiments, a pharmaceutical composition may be for administration *in vivo*. A mammal includes any mammal, such as by way of non-limitimg example, cattle, pigs, sheep, goats, horses, camels, buffalo, cats, dogs, rats, mice, and humans. A highly preferred subject mammal is a human.

In an embodiment, the pharmaceutical compositions may be formulated with pharmaceutically acceptable excipients such as carriers, solvents, stabilizers, adjuvants, diluents, etc., depending upon the particular mode of administration and dosage form. The pharmaceutical compositions should generally be formulated to achieve a physiologically compatible pH, and may range from a pH of about 3 to a pH of about 11, preferably about pH 3 to about pH 7, depending on the formulation and route of administration. In alternative embodiments, it may be preferred that the pH is adjusted to a range from about pH 5.0 to about pH 8. More particularly, the pharmaceutical compositions may comprise a therapeutically or prophylactically effective amount of at least one compound as described herein, together with one or more pharmaceutically acceptable excipients. Optionally, the pharmaceutical compositions may comprise a combination of the compounds described herein, or may include a second active ingredient useful in the treatment or prevention of bacterial infection (e.g., anti-bacterial or anti-microbial agents).

Formulations, e.g., for parenteral or oral administration, are most typically solids, liquid solutions, emulsions or suspensions, while inhalable formulations for pulmonary administration are generally liquids or powders, with powder formulations being generally preferred. A preferred pharmaceutical composition may also be formulated as a lyophilized solid that is reconstituted with a physiologically compatible solvent prior to administration. Alternative pharmaceutical compositions may be formulated as syrups, creams, ointments, tablets, capsules and the like.

The term "pharmaceutically acceptable excipient" refers to an excipient for administration of a pharmaceutical agent, such as the compounds described herein. The term refers to any pharmaceutical excipient that may be administered without undue toxicity. Pharmaceutically acceptable excipients may include, for example, inactive ingredients such as disintegrators, binders, fillers, and lubricants used in formulating pharmaceutical products.

Pharmaceutically acceptable excipients are determined in part by the particular composition being administered, as well as by the particular method used to administer the composition. Accordingly, there exists a wide variety of suitable formulations of pharmaceutical compositions (see, e.g., Remington's Pharmaceutical Sciences).

Suitable excipients may be carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants such as ascorbic acid; chelating agents such as EDTA; carbohydrates such as dextrin, hydroxyalkylcellulose, hydroxyalkylmethylcellulose, stearic acid; liquids such as oils, water, saline, glycerol and ethanol; wetting or emulsifying agents; pH buffering substances; and the like. Liposomes are also included within the definition of pharmaceutically acceptable excipients.

Disintegrator include, for example, agar-agar, algins, calcium carbonate, carboxmethylcellulose, cellulose, clays, colloid silicon dioxide, croscarmellose sodium, crospovidone, gums, magnesium aluminium silicate, methylcellulose, polacrilin potassium, sodium alginate, low substituted hydroxypropylcellulose, and cross-linked polyvinylpyrrolidone hydroxypropylcellulose, sodium starch glycolate, and starch.

Binders include, for example, microcrystalline cellulose, hydroxymethyl cellulose, hydroxypropylcellulose, and polyvinylpyrrolidone.

Fillers include, for example, calcium carbonate, calcium phosphate, dibasic calcium phosphate, tribasic calcium sulfate, calcium carboxymethylcellulose, cellulose, dextrin derivatives, dextrin, dextrose, fructose, lactitol, lactose, magnesium carbonate, magnesium oxide, maltitol, maltodextrins, maltose, sorbitol, starch, sucrose, sugar, and xylitol.

Lubricants include, for example, agar, calcium stearate, ethyl oleate, ethyl laureate, glycerin, glyceryl palmitostearate, hydrogenated vegetable oil, magnesium oxide, magnesium stearate, mannitol, poloxamer, glycols, sodium benzoate, sodium lauryl sulfate, sodium stearyl, sorbitol, stearic acid, talc, and zinc stearate.

The pharmaceutical compositions described herein may be formulated in any form suitable for the intended method of administration. When intended for oral use for example, tablets, troches, lozenges, aqueous or oil suspensions, non-aqueous solutions, dispersible powders or granules (including micronized particles or nanoparticles), emulsions, hard or soft capsules, syrups or elixirs may be prepared. Compositions intended for oral use may be prepared according to any method known to the art for the manufacture of pharmaceutical compositions, and such compositions may contain one or more agents including sweetening agents, flavoring agents, coloring agents and preserving agents, in order to provide a palatable preparation.

Pharmaceutically acceptable excipients particularly suitable for use in conjunction with tablets include, for example, inert diluents, such as celluloses, calcium or sodium carbonate, lactose, calcium or sodium phosphate; disintegrating agents, such as cross-linked povidone, maize starch, or alginic acid; binding agents, such as povidone, starch, gelatin or acacia; and lubricating agents, such as magnesium stearate, stearic acid or talc.

Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed. To those skilled in the pharmaceutical research and manufacturing, it is generally known that tablet formulations permit generous additions of inactive ingredients including excipients and coating substances, and a high percentage of fillers. However, the addition of inactive ingredients may limit the amount of active ingredients carried in each tablet.

Formulations for oral use may be also presented as hard gelatin capsules where the active ingredient is mixed with an inert solid diluent, for example celluloses, lactose, calcium phosphate or kaolin, or as soft gelatin capsules wherein the active ingredient is mixed with non-aqueous or oil medium, such as glycerin, propylene glycol, polyethylene glycol, peanut oil, liquid paraffin or olive oil. Capsules may allow for inclusion of a larger amount of binders, instead of fillers as used more in tablets. In one embodiment, by weight, 2-10% of the capsule is disintegrator, 2-30% is binder, 2-30% is filler, and 0.3-0.8% is lubricant. A multitude of substances may be suitably included as disintegrator, binder, filler, and lubricant. One example is to use magnesium stearate as lubricant, microcrystalline cellulose as binder, and croscarmellose as disintegrator. In one embodiment, the capsule formulation further includes povidone. By weight povidone may constitute 1-4% of the capsule. The capsule shell may be made of hard gelatin in one embodiment. The shell may be clear or opaque, white or with color in various embodiments. In one embodiment, the capsule is size 1. Other sizes may be adopted in alternative embodiments.

In another embodiment, pharmaceutical compositions may be formulated as suspensions comprising a compound of the embodiments in admixture with at least one pharmaceutically acceptable excipient suitable for the manufacture of a suspension.

In yet another embodiment, pharmaceutical compositions may be formulated as dispersible powders and granules suitable for preparation of a suspension by the addition of suitable excipients.

Excipients suitable for use in connection with suspensions include suspending agents, such as sodium carboxymethylcellulose, methylcellulose, hydroxypropyl methylcellulose, sodium alginate, polyvinylpyrrolidone, gum tragacanth, gum acacia, dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycethanol), a condensation product of ethylene oxide with a partial ester derived from a fatty acid and a hexitol anhydride (e.g., polyoxyethylene sorbitan monooleate); and thickening agents, such as carbomer, beeswax, hard paraffin or cetyl alcohol. The suspensions may also contain one or more preservatives such as acetic acid, methyl and/or n-propyl p-hydroxy-benzoate; one or more coloring agents; one or more flavoring agents; and one or more sweetening agents such as sucrose or saccharin.

The pharmaceutical compositions may also be in the form of oil-in water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth; naturally occurring phosphatides, such as soybean lecithin, esters or partial esters derived from fatty acids; hexitol anhydrides, such as sorbitan monooleate; and condensation products of these partial esters with ethylene oxide, such as polyoxyethylene sorbitan monooleate. The emulsion may also contain sweetening and flavoring agents. Syrups and elixirs may be formulated with sweetening agents, such as glycerol, sorbitol or sucrose. Such formulations may also contain a demulcent, a preservative, a flavoring or a coloring agent.

Additionally, the pharmaceutical compositions may be in the form of a sterile injectable preparation, such as a sterile injectable aqueous emulsion or oleaginous suspension. This emulsion or suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent, such as a solution in 1,2-propane-diol.

The sterile injectable preparation may also be prepared as a lyophilized powder. Among the acceptable vehicles and solvents that may be employed are water, Ringer's solution, and isotonic sodium chloride solution. In addition, sterile fixed oils may be employed as a solvent or suspending medium. For this purpose any bland fixed oil may be employed including synthetic mono- or diglycerides. In addition, fatty acids such as oleic acid may likewise be used in the preparation of injectables.

To obtain a stable water-soluble dose form of a pharmaceutical composition, a pharmaceutically acceptable salt of a compound described herein may be dissolved in an aqueous solution of an organic or inorganic acid, such as 0.3 M solution of succinic acid, or more preferably, citric acid. If a soluble salt form is not available, the compound may be dissolved in a suitable co-solvent or combination of co-solvents. Examples of suitable co-solvents include alcohol, propylene glycol, polyethylene glycol 300, polysorbate 80, glycerin and the like in concentrations ranging from about 0 to about 60% of the total volume. In one embodiment, the active compound is dissolved in DMSO and diluted with water.

The pharmaceutical composition may also be in the form of a solution of a salt form of the active ingredient in an appropriate aqueous vehicle, such as water or isotonic saline or dextrose solution. Also contemplated are compounds which have been modified by substitutions or additions of chemical or biochemical moieties which make them more suitable for delivery (e.g., increase solubility, bioactivity, palatability, decrease adverse reactions, etc.), for example by esterification, glycosylation, PEGylation, etc.

In a preferred embodiment, the compounds described herein may be formulated for oral administration in a lipid-based formulation suitable for low solubility compounds. Lipid-based formulations may generally enhance the oral bioavailability of such compounds.

As such, a preferred pharmaceutical composition comprises a therapeutically or prophylactically effective amount of a compound described herein, together with at least one pharmaceutically acceptable excipient selected from the group consisting of-medium chain fatty acids or propylene glycol esters thereof (e.g., propylene glycol esters of edible fatty acids such as caprylic and capric fatty acids) and pharmaceutically acceptable surfactants such as polyoxyl 40 hydrogenated castor oil.

In an alternative preferred embodiment, cyclodextrins may be added as aqueous solubility enhancers. Preferred cyclodextrins include hydroxypropyl, hydroxyethyl, glucosyl, maltosyl and maltotriosyl derivatives of α-, β-, and γ-cyclodextrin. A particularly preferred cyclodextrin solubility enhancer is hydroxypropyl-o-cyclodextrin (BPBC), which may be added to any of the above-described compositions to further improve the aqueous solubility characteristics of the compounds of the embodiments. In one embodiment, the composition comprises about 0.1% to about 20% hydroxypropyl-o-cyclodextrin, more preferably about 1% to about 15% hydroxypropyl-o-cyclodextrin, and even more preferably from about 2.5% to about 10% hydroxypropyl-o-cyclodextrin. The amount of solubility enhancer employed will depend on the amount of the compound of the embodiments in the composition.

A pharmaceutical composition preferably contains a total amount of the active ingredient(s) sufficient to achieve an intended therapeutic effect. More specifically, in some embodiments, the pharmaceutical composition contains a therapeutically effective amount (e.g., an amount of pirfenidone and a toll-like receptor agonist compound that is effective in the prevention or treatment of neutropenia). The total amounts of the compound that may be combined with the carrier materials to produce a unitary dosing form will vary depending upon the host treated and the particular mode of administration. Preferably, the compositions are formulated so that a dose of between 0.01 to 100 mg/kg body weight/day of each of pirfenidone and a toll-like receptor agonist compound is administered to a subject receiving the compositions.

The term "unit dosage form", as used herein, refers to physically discrete units suitable as unitary dosages for human and animal subjects, each unit containing a predetermined quantity of compounds of the invention calculated in an amount sufficient to produce the desired effect in association with a pharmaceutically acceptable diluent, carrier or vehicle. The specifications for the novel unit dosage forms of the present invention depend on the particular compounds employed and the effect to be achieved, and the pharmacodynamics associated with each compound in the host Unit dosage forms for injection or intravenous administration may comprise the compound of the present invention in a composition as a soluble in sterile water, normal saline or another pharmaceutically acceptable carrier.

It is to be understood that the description, specific examples and data, while indicating exemplary embodiments, are given by way of illustration and are not intended to limit the various embodiments of the present disclosure.

### EXAMPLES

### EXAMPLE 1

Human PBMCs (10⁵ cells per well) were pretreated with pirfenidone (5 mM to 5 µM) for one hour in a 96-well plate and then stimulated with LPS (1µg/ml to 0.01 ng/ml) for 1, 2, 8, or 24 hours at 37°C. Subsequently, cells were spun and supernatants collected and assayed for protein expression using the BioRad Multiplex Cytokine Platform, which enables 17 different cytokines to be analyzed simultaneously: G-CSF, GM-CSF, IFN-γ, IL-1β, IL-2, IL-4, IL-5, IL-6, IL-7, IL-8, IL-10, IL-12 (p70), IL-13, IL-17, MCP-1, MIP-1β, and TNF-α.

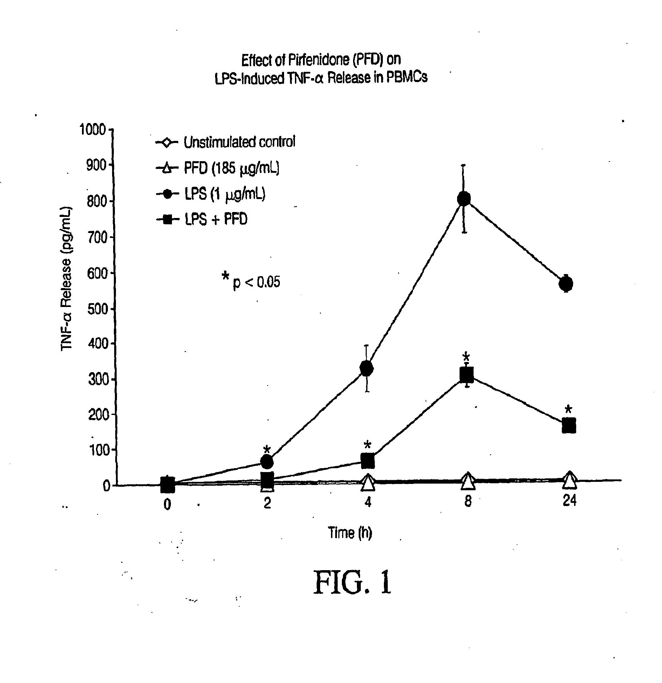

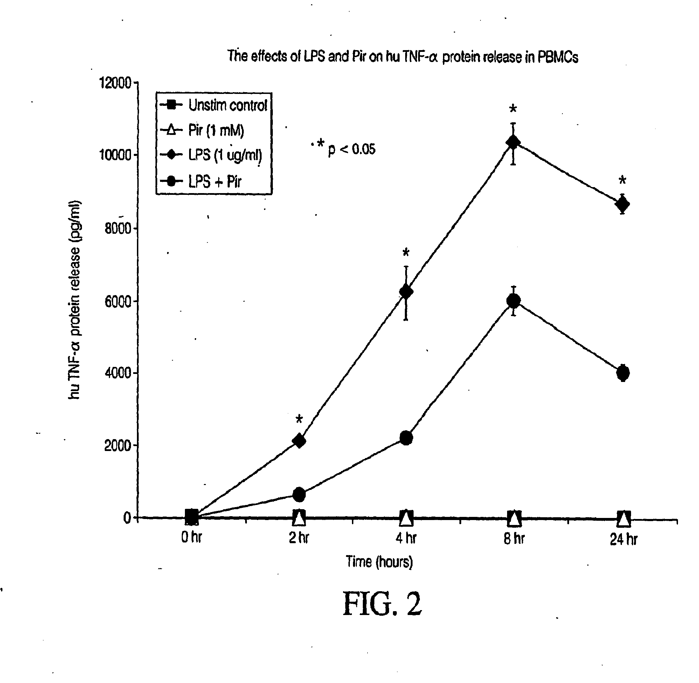

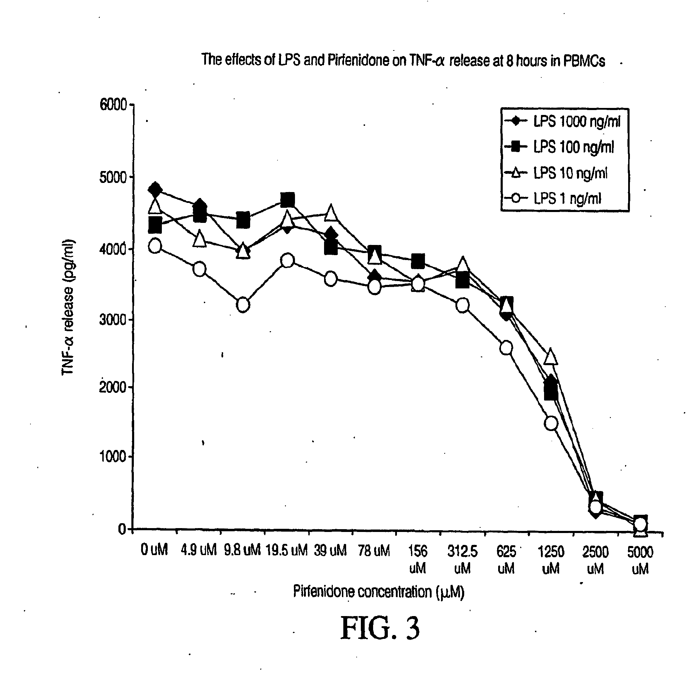

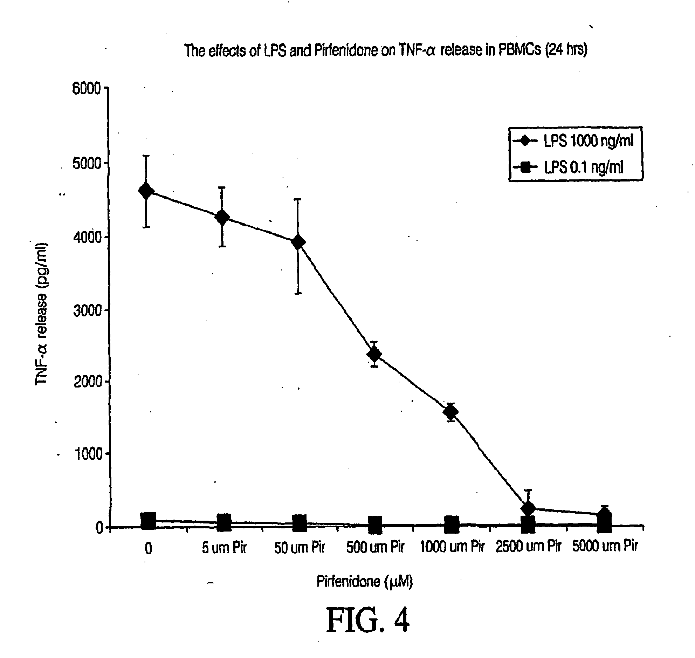

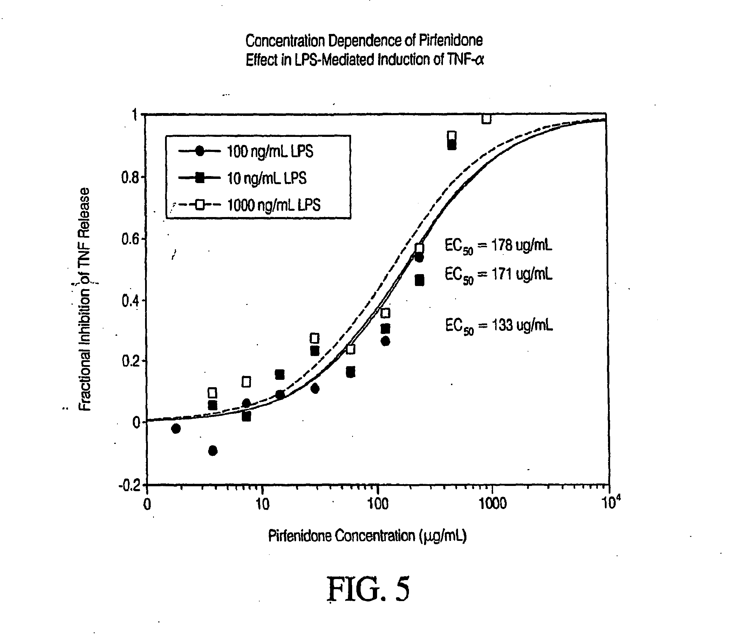

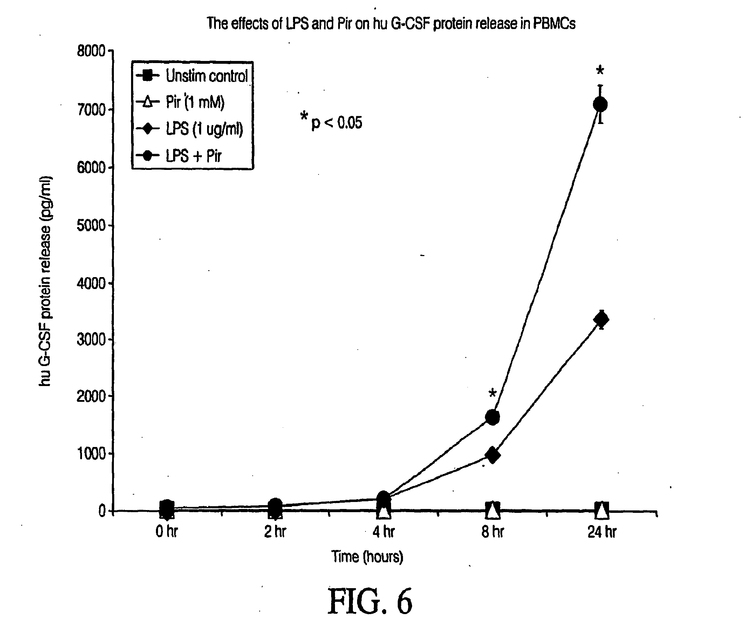

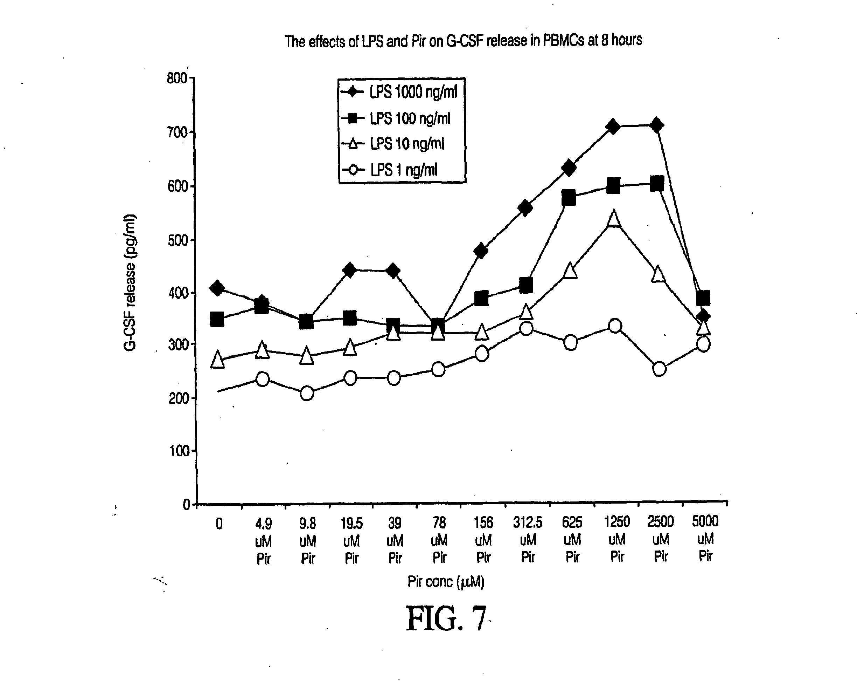

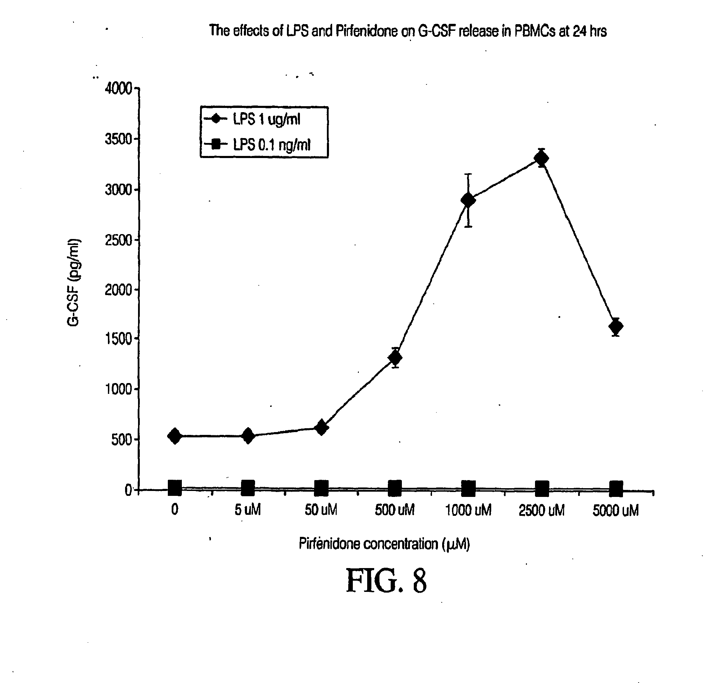

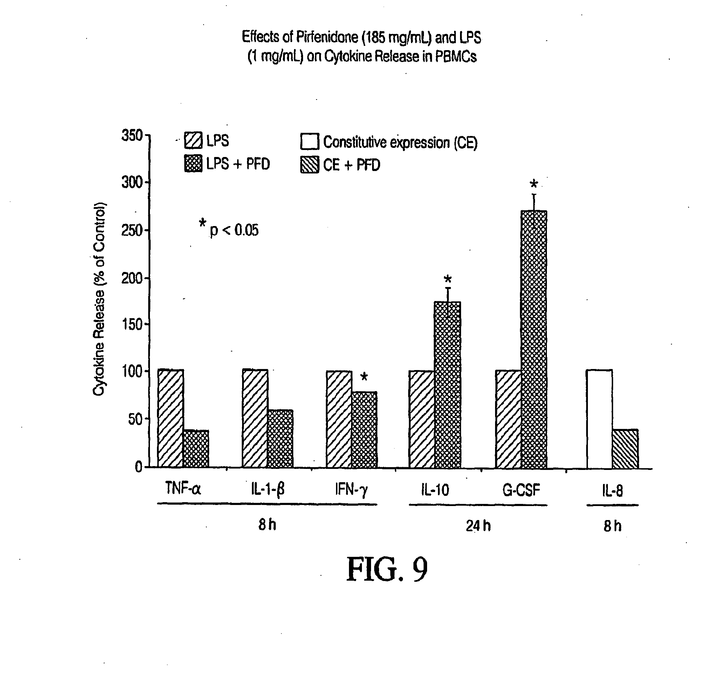

Figure 1 shows that pirfenidone (185 µg/mL) and LPS (1µg/mL) reduced TNF- α expression in PBMCs. Figure 2 shows the effects of pirfenidone (inM) and LPS (1 µg/mL) on TNF-α release in PBMCs. Figure 3 shows the effects of LPS and pirfenidone at varying concentrations (0-5,000µM) on TNF-α release at 8 hours in PBMCs. Figure 4 shows the effects of LPS (1,000 ng/mL or 0.1 ng/mL) and pirfenidone at varying concentrations (0-5,000µM) on TNF-α release at 24 hours in PBMCs. Figure 5 shows the concentration dependence of pirfenidone effect in LPS-mediated induction of TNF-α. Figure 6 shows the effects of LPS (1 µg/ml) and pirfenidone (1 µM) on G-CSF release in PBMCs at 0, 2, 4, 8, and 24 hours. Figure 7 shows the effects of LPS (1,000 ng/mL, 100 ng/mL, 10 ng/mL, or 1 ng/mL) and pirfenidone at varying concentrations (0-5,000µM) on G-CSF release in PBMCs at 8 hours. Figure 8 shows the effects of LPS (1 µg/ml or 0.1 ng/ml) and varying concentrations of pirfenidone (0-5,000 µM) on G-CSF release in PMBCs at 24 hours. Figure 9 shows the effects of pirfenidone (185 mg/mL) and LPS (1 mg/mL) on cytokine release in PBMCs. Notably, TNF-α release is decreased and G-CSF release is increased.

Pirfenidone had differential effects on LPS-mediated cytokine induction in PBMCs. Optimal expression of the pro-inflammatory cytokines IL-1β and TNF-α was observed at 8 hours, while the pirfenidone IC₅₀s for these cytokines were approximately 2.5 and 1 mM, respectively. In contrast, LPS-induced expression of IL-10 and G-CSF were augmented in 'the presence of pirfenidone (1mM) by 35% and 100%, respectively. Pirfenidone inhibited LPS-mediated release of TNF- a, IFN-γ, IL-1β, and GM-CSF and augmented LPS-mediated release of G-CSF, IL-10 and MCP-1. Pirfenidone inhibited constitutive (but not LPS-mediated) expression of IL-6, IL-8, and MIP-1β. LPS did not induce expression of IL-2, IL-4, IL-5, IL-7, IL-12, IL-13, or IL-17.

### EXAMPLE 2

The effect of pirfenidone and various TLR agonists on TNF-α and G-CSF release in PBMCs was examined. Briefly, human PBMCs (10⁵ cells per well) were pretreated with pirfenidone (185 mg/mL) for one hour in a 96-well plate and then stimulated with lipopolysaccharide (LPS, binds TLR4), Fibrin (binds TLR4), lipoteichoic acid (LTA, binds TLR2), peptidoglycan (PG, binds TLR2), or CpG (bacterial DNA, binds TLR9) for 24 hours at 37°C. Subsequently, cells were spun and supernatants collected and assayed for TNF-α and G-CSF expression.

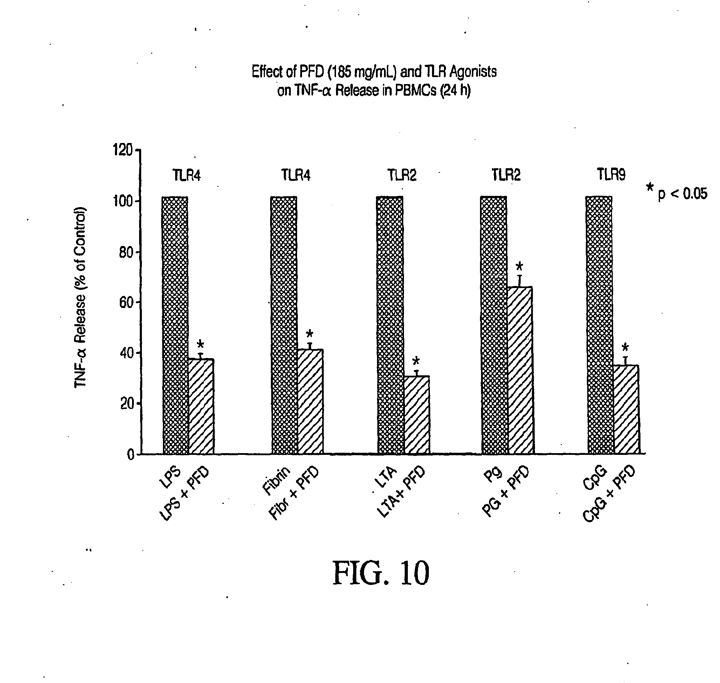

Figure 10 shows that TNF-α release is inhibited for all TLR agonists.

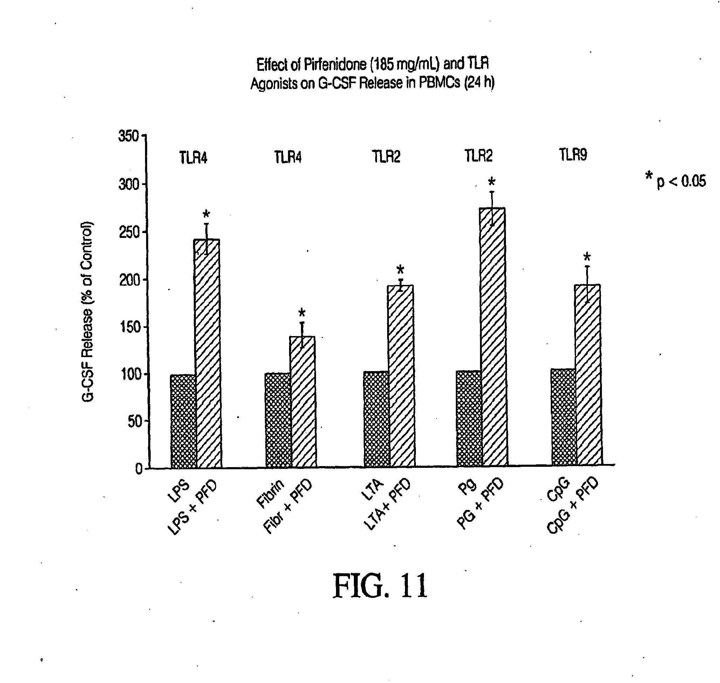

Figure 11 shows that G-CSF release was augmented for all TLR agonists.

### EXAMPLE 3

The effect of various p38 inhibitors on LPS-mediated TNF- a and G-CSF release from human PBMCs was examined. Briefly, human PBMCs (10⁵ cells per well) were pretreated with pirfenidone, a hydroxyl-pirfenidone derivative, SB202190 (commercially available p38 inhibitor), or SB 203580 (commercially available p38 inhibitor) for one hour in a 96-well plate and then stimulated with lipopolysaccharide (LPS, binds TLR4), Fibrin (binds TLR4), lipoteichoic acid (LTA, binds TLR2), peptidoglycan (PG, binds TLR2), or CpG (bacterial DNA, binds TLR9) for 24 hours at 37°C. Subsequently, cells were spun and supernatants collected and assayed for G-CSF expression.

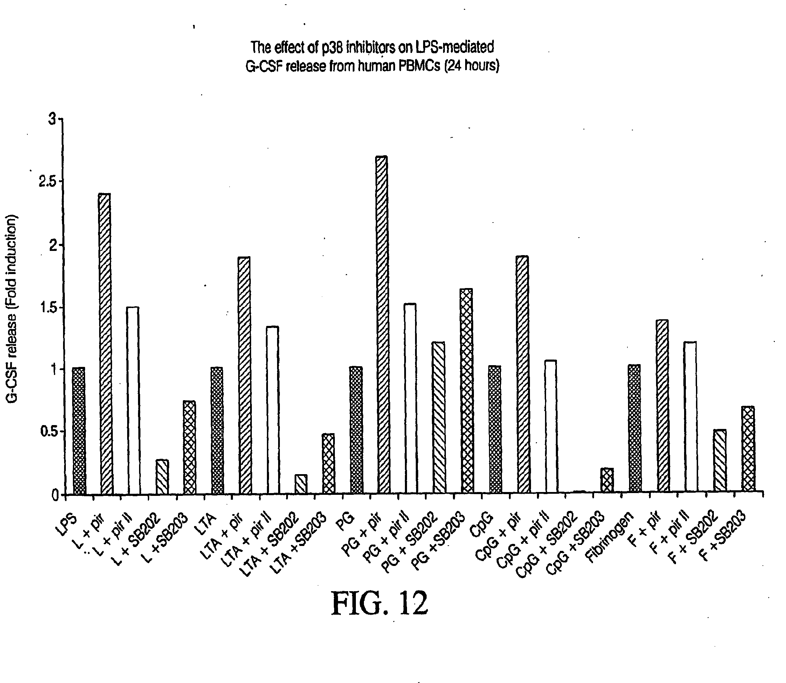

All of the p38 inhibitors blocked TLR-agonist stimulation of PBMCs and release of TNF- a. Figure 12 shows the results of the experiment as fold induction. Each TLR agonist alone is set to 1 (positive control) and the subsequent release of G-CSF in the presence of p38 inhibitors is determined relative to the positive control. Notably, the augmented effects of LPS and pirfenidone are not unique to LPS stimulation, but are unique to pirfenidone as none of the other p38 inhibitors augmented TLR-induced G-CSF release, except for PG.

### EXAMPLE 4

The effect of pirfenidone and various TLR agonists on TNF-α and G-CSF release in PBMCs is examined. Briefly, human PBMCs (10⁵ cells per well) are pretreated with pirfenidone (185 mg/mL) for one hour in a 96-well plate and then stimulated with TLR7 agonists: 7-thia-8-oxoguanosine (TOG or isatoribine), 7-deazaguanosine, 7-allyl-8-oxoguanosine (loxoribine), 7-dezaguanosine (7-deza-G), imiquimod (R837), or R848 for 24 hours at 37°C. Subsequently, cells are spun and supernatants collected and assayed for TNF-a and G-CSF expression.

TNF-α release is inhibited for all TLR7 agonists and G-CSF release is augmented for all TLR7 agonists.

### EXAMPLE 5

Neutropenia is induced in mice by administering a chemotherapeutic agent on day 0 at a dose optimized to give a maximum reduction in neutrophils on day 8 without permanently damaging the mouse's ability to recover. Mice are administered a composition comprising pirfenidone and 7-thio-8-oxoguanosine (TOG) or a placebo on days 1-6 and sacrificed by terminal bleed on day 8. A complete blood count (CBC), including absolute neutrophil count (ANC) is run on the blood samples.

Absolute neutrophils in mice receiving pirfenidone and 7-thio-8-oxoguanosine (TOG) are approximately normal, while absolute neutrophils in mice receiving placebo are on average, dangerously low.

### EXAMPLE 6

Neutropenia is induced in mice by exposing them to controlled doses of radiation from a cesium source. The dose is optimized to give a maximum reduction in neutrophils on day 8 without permanently suppressing neutrophil counts. Mice are administered a composition comprising pirfenidone and 7-dezaguanosine or placebo on days 1-6 and sacrificed by terminal bleed on day 8. A CBC is run on the blood samples.

Absolute neutrophils in mice receiving pirfenidone and 7-dezaguanosine are approximately normal. Mice receiving placebo suffer from severe neutropenia.

### EXAMPLE 7

Gray collie dogs discovered by the University of Tennessee to be a naturally-occurring animal model for the study of cyclic neutropenia are divided into three groups: two treated groups and one placebo group. Blood samples are taken daily to determine the ANC. Dogs in treated Group A are administered an effective dose of a composition comprising pirfenidone and imiquimod (a TLR7 agonist) once neutrophil counts drop below 1,000 cells/mL. Treatment is continued for 3 days. Dogs in treated Group B are administered an effective dose of a composition comprising pirfenidone and imiquimod for 16 weeks.

Neutrophil counts in dogs in treatment group A return to normal within 2 days compared to neutrophil counts in dogs in the placebo group which return to normal in 6 days. Dogs in Group B do not experience low neutrophil counts during the course of the study. Dogs in the placebo group experience severe neutropenia every 3 weeks for an average of 6 days at a time throughout the study.

### EXAMPLE 8

Patients diagnosed with neutropenia participate in a double-blind, placebo controlled, randomized study to provide insight into the treatment of neutropenia using compositions comprising pirfenidone and loxoribine. The diagnosis of neutropenia is confirmed by a complete blood count. For the purposes of this study, neutrophil counts of 1,000-1,500 cells/mL is considered a condition of mild neutropenia. A count of 1,000 cells to 500 cells/mL is moderate neutropenia and a count of 500 cells/mL or less is severe neutropenia. Patients are randomly assigned into treatment compound or placebo using a modified permuted-block randomization method.

Because the most common cause of neutropenia is cancer chemotherapy, the principal inclusion criteria is subjects recently diagnosed of chemotherapy sensitive malignancies and who are willing to undergo multiple cycles of chemotherapy regimen.

Patients are separated into two groups. The test group receives a composition comprising pirfenidone and loxoribine. Test group patients receive oral tablets (treatment compound or placebo) at a dose of 400 mg three times a day for the course of the study 12 weeks. The test group patients are also administered chemotherapy in three cycles of one month each. The first dose is administered before the chemotherapy dosing session; either the same day or 48 hours prior.

The patients of the control group do not receive any doses of the test composition and receive only chemotherapy drugs. Ten test group patients are matched with 12 control patients for drug regimen, cancer types and age.

Routine laboratory investigations are performed at initiation and at several points during the study period. Clinical evaluation and complete blood counts (CBC) are performed twice a week.

Results are analyzed to study the severity, incidence and duration of neutropenia in each group. Severity of neutropenia is measured by calculating the neutropenia ratio - the minimum neutrophil count attained during chemotherapy over the predosing value. Subjects receiving a composition comprising pirfenidone and loxoribine exhibit mild or no neutropenia. All of the control subjects exhibit symptoms of neutropenia by the third cycle. Neutropenia ratios are significantly lower in control groups as compared to test group in all the three cycles of chemotherapy. The incidence of moderate to severe neutropenia and the duration of neutropenia is higher in control group as compared to the test group.

## Claims

1. A therapeutically effective amount of pirfenidone and one or more toll-like receptor (TLR) agonists, for use in treating or inhibiting neutropenia in a subject in need thereof.

2. A therapeutically effective amount of pirfenidone, for use in treating or inhibiting neutropenia in a subject in need thereof, wherein said subject has been administered one or more toll-like receptor (TLR) agonists.

3. One or more toll-like receptor (TLR) agonists, for use in treating or inhibiting neutropenia in a subject in need thereof, wherein said subject has been administered a therapeutically effective amount of pirfenidone.

4. The use of a therapeutically effective amount of pirfenidone and one or more toll-like receptor (TLR) agonists in the manufacture of a medicament for treating or inhibiting neutropenia in a subject in need thereof.

5. The use of a therapeutically effective amount of pirfenidone in the manufacture of a medicament for treating or inhibiting neutropenia in a subject in need thereof, wherein said subject has been administered one or more toll-like receptor (TLR) agonists.

6. The use of one or more toll-like receptor (TLR) agonists in the manufacture of a medicament for treating or inhibiting neutropenia in a subject in need thereof, wherein said subject has been administered a therapeutically effective amount of pirfenidone.

7. The pirfenidone or one or more TLR agonists for use of Claim 1, or use of Claim 4, wherein said pirfenidone and said one or more TLR agonists are for administration simultaneously.

8. The pirfenidone or one or more TLR agonists for use of Claim 1, or use of Claim 4, wherein said pirfenidone and said one or more TLR agonists are for delivery as separate formulations.

9. The pirfenidone or one or more TLR agonists for use of Claim 1, or use of Claim 4, wherein said pirfenidone and said one or more TLR agonists are co-formulated.

10. The pirfenidone or one or more TLR agonists for use of claims 1, 2 or 3 or the use of Claims 4, 5 or 6, wherein the subject is a human.

11. The pirfenidone or one or more TLR agonists for use of Claims 1, 2, 3, 7, 8, 9 or 10, the use of Claims 4, 5, 6 or 10, wherein the pirfenidone or one or more TLR agonists, or medicament is for administration in an amount effective for increasing the number of neutrophils in the subject.

12. The pirfenidone or one or more TLR agonists for use of Claims 1,2, 3, 7, 8, 9 or 11, or the use of Claims 4, 5, 6 or 11, wherein the therapeutically effective amount is less than 50% of an amount that causes an undesirable side effect in the subject.

13. The pirfenidone or one or more TLR agonists for use of Claims 1, 2 or 3, or the use of Claims 4, 5 or 6, wherein the pirfenidone or one or more TLR agonists or medicament is for oral administration.

14. The pirfenidone or one or more TLR agonists for use of Claims 1, 2 or 3 or the use of Claims 4, 5 or 6, wherein the pirfenidone or one or more TLR agonists or medicament is for administration twice per day.

15. The pirfenidone or one or more TLR agonists for use of Claims 1, 2 or 3 or the use of Claims 4, 5 or 6, wherein the pirfenidone or one or more TLR agonists or medicamentis for administration three times per day.

16. The pirfenidone for use of Claims 1 or 2, or the use of Claims 4 or 5 , wherein the pirfenidone, or a medicament is for providing the pirfenidone in a dose of from about 100 to about 400 milligrams.

17. The pirfenidone for use of Claims 1 or 2 or the use of Claims 4 or 5 wherein the pirfenidone or medicament is or administering the pirfenidone such that the daily intake is from about 800 to about 4000 mg/day.

18. The pirfenidone for use of Claims 1. or 2 or the use of Claims 4 or 5, wherein the pirfenidone or medicament is for administering the pirfenidone such that the daily intake is about 1200mg/day or higher.

19. The pirfenidone or one or more TLR agonists for use of Claims 1, 2 or 3 or the use of Claims 4, 5 or 6, wherein said neutropenia is severe neutropenia.

20. The pirfenidone or one or more TLR agonists for use of Claims 1, 2 or 3 or the use of Claims 4, 5 or 6, wherein said neutropenia is selected from the group consisting of neutropenia associated with chemotherapy, neutropenia associated with conventional oncology therapy, drug-induced neutropenia, disease-induced neutropenia, genetic neutropenia, toxin-induced neutropenia, congenital neutropenia, cyclic neutropenia, idiopathic neutropenia, and radiation-induced neutropenia.

21. The pirfenidone or one or more TLR agonists for use of Claims 1, 2 or 3, or the use of Claims 4, 5 or 6, wherein the one or more TLR agonists comprises at least one TLR 7 agonist,

22. The pirfenidone or one or more TLR agonists for use, or the use of Claim 21, wherein the TLR 7 agonist is selected from the group consisting of 7-thia-8-oxoguanosine, 7-deazaguanosine, 7-allyl-8-oxoguanosine, 7-dezaguanosine, imiquimod, and R848.

23. A kit of parts comprising:
a therapeutically effective amount of pirfenidone; and
one or more toll-like receptor (TLR) agonists; for use in treating or inhibiting neutropenia in a subject in need thereof, wherein the therapeutically effective amount of pirfenidone and the one or more toll-like receptor (TLR) agonists are provided in separate formulations.

## Patentansprüche

1. Therapeutisch wirksame Menge von Pirfenidon und mindestens einem Agonisten eines toll-artigen ("Toll-like") Rezeptors (TLR) für die Verwendung beim Behandeln oder Hemmen von Neutropenie bei einem Individuum, das diese(s) benötigt.

2. Therapeutisch wirksame Menge von Pirfenidon für die Verwendung beim Behandeln oder Hemmen von Neutropenie bei einem Individuum, das diese(s) benötigt, wobei dem Individuum mindestens ein Agonist eines toll-artigen Rezeptors (TLR) verabreicht worden ist.

3. Mindestens ein Agonist eines toll-artigen Rezeptors (TLR) für die Verwendung beim Behandeln oder Hemmen von Neutropenie bei einem Individuum, das diese(s) benötigt, wobei dem Individuum eine therapeutisch wirksame Menge von Pirfenidon verabreicht worden ist.

4. Verwendung einer therapeutisch wirksamen Menge von Pirfenidon und mindestens einem Agonisten eines toll-artigen ("Toll-like") Rezeptors (TLR) bei der Herstellung eines Medikaments zum Behandeln oder Hemmen von Neutropenie bei einem Individuum, das diese(s) benötigt.

5. Verwendung einer therapeutisch wirksamen Menge von Pirfenidon bei der Herstellung eines Medikaments zum Behandeln oder Hemmen von Neutropenie bei einem Individuum, das diese(s) benötigt, wobei dem Individuum mindestens ein Agonist eines toll-artigen Rezeptors (TLR) verabreicht worden ist.

6. Verwendung von mindestens einem Agonisten eines toll-artigen ("Toll-like") Rezeptors (TLR) bei der Herstellung eines Medikaments zum Behandeln oder Hemmen von Neutropenie bei einem Individuum, das diese(s) benötigt, wobei dem Individuum eine therapeutisch wirksame Menge von Pirfenidon verabreicht worden ist.

7. Pirfenidon oder mindestens ein TLR-Agonist zur Verwendung nach Anspruch 1 oder Verwendung nach Anspruch 4, wobei das Pirfenidon und der mindestens eine TLR-Agonist der gleichzeitigen Verabreichung dienen.

8. Pirfenidon oder mindestens ein TLR-Agonist zur Verwendung nach Anspruch 1 oder Verwendung nach Anspruch 4, wobei das Pirfenidon und der mindestens eine TLR-Agonist der Abgabe als separate Formulierungen dienen.

9. Pirfenidon oder mindestens ein TLR-Agonist zur Verwendung nach Anspruch 1 oder Verwendung nach Anspruch 4, wobei das Pirfenidon und der mindestens eine TLR-Agonist coformuliert sind.

10. Pirfenidon oder mindestens ein TLR-Agonist zur Verwendung nach Anspruch 1, 2 oder 3 oder die Verwendung nach Anspruch 4, 5 oder 6, wobei es sich bei dem Individuum um einen Menschen handelt.

11. Pirfenidon oder mindestens ein TLR-Agonist zur Verwendung nach Anspruch 1, 2, 3, 7, 8, 9 oder 10 oder die Verwendung nach Anspruch 4, 5, 6, oder 10, wobei das Pirfenidon und mindestens ein TLR-Agonist oder das Medikament der Verabreichung in einer Menge dienen, die zum Erhöhen der Anzahl von Neutrophilen in dem Individuum wirksam ist.

12. Pirfenidon oder mindestens ein TLR-Agonist zur Verwendung nach Anspruch 1, 2, 3, 7, 8, 9 oder 11 oder die Verwendung nach Anspruch 4, 5, 6, oder 11, wobei die therapeutisch wirksame Menge weniger als 50 % einer Menge beträgt, die eine unerwünschte Nebenwirkung in dem Individuum hervorruft.

13. Pirfenidon oder mindestens ein TLR-Agonist zur Verwendung nach Anspruch 1, 2 oder 3 oder die Verwendung nach Anspruch 4, 5 oder 6, wobei das Pirfenidon oder mindestens ein TLR-Agonist oder Medikament der oralen Verabreichung dient.

14. Pirfenidon oder mindestens ein TLR-Agonist zur Verwendung nach Anspruch 1, 2 oder 3 oder die Verwendung nach Anspruch 4, 5 oder 6, wobei das Pirfenidon oder mindestens ein TLR-Agonist oder Medikament der Verabreichung zweimal täglich dient.

15. Pirfenidon oder mindestens ein TLR-Agonist zur Verwendung nach Anspruch 1, 2 oder 3 oder die Verwendung nach Anspruch 4, 5 oder 6, wobei das Pirfenidon oder mindestens ein TLR-Agonist oder Medikament der Verabreichung dreimal täglich dient.

16. Pirfenidon zur Verwendung nach Anspruch 1 oder 2 oder die Verwendung nach Anspruch 4 oder 5, wobei das Pirfenidon oder Medikament zum Vorsehen des Pirfenidons in einer Dosis von etwa 100 bis etwa 400 Milligramm dient.

17. Pirfenidon zur Verwendung nach Anspruch 1 oder 2 oder die Verwendung nach Anspruch 4 oder 5, wobei das Pirfenidon oder Medikament zum Verabreichen des Pirfenidons derart dient, dass die tägliche Aufnahme von etwa 800 bis etwa 400 mg/Tag beträgt.

18. Pirfenidon zur Verwendung nach Anspruch 1 oder 2 oder die Verwendung nach Anspruch 4 oder 5, wobei das Pirfenidon oder Medikament zum Verabreichen des Pirfenidons derart dient, dass die tägliche Aufnahme etwa 1200 mg/Tag oder höher beträgt.

19. Pirfenidon oder mindestens ein TLR-Agonist zur Verwendung nach Anspruch 1, 2 oder 3 oder die Verwendung nach Anspruch 4, 5 oder 6, wobei es sich bei der Neutropenie um schwere Neutropenie handelt.

20. Pirfenidon oder mindestens ein TLR-Agonist zur Verwendung nach Anspruch 1, 2 oder 3 oder die Verwendung nach Anspruch 4, 5 oder 6, wobei die Neutropenie aus der Gruppe ausgewählt ist, die aus Neutropenie in Zusammenhang mit Chemotherapie, Neutropenie in Zusammenhang mit einer konventionellen Onkologietherapie, arzneimittelinduzierter Neutropenie, krankheitsinduzierter Neutropenie, genetischer Neutropenie, toxininduzierter Neutropenie, kongenitaler Neutropenie, zyklischer Neutropenie, idiopathischer Neutropenie und strahlungsinduzierter Neutropenie besteht.

21. Pirfenidon oder mindestens ein TLR-Agonist zur Verwendung nach Anspruch 1, 2 oder 3 oder die Verwendung nach Anspruch 4, 5 oder 6, wobei der mindestens eine TLR-Agonist mindestens einen TLR7-Agonisten umfasst.

22. Pirfenidon oder mindestens ein TLR-Agonist zur Verwendung oder die Verwendung nach Anspruch 21, wobei der TLR7-Agonist aus der Gruppe ausgewählt ist, die aus 7-Thia-8-oxoguanosin, 7-Deazaguanosin, 7-Allyl-8-oxoguanosin, 7-Deazaguanosin, Imiquimod und R848 besteht.

23. Kit von Teilen, welches:
eine therapeutisch wirksame Menge von Pirfenidon und
mindestens einen Agonisten eines toll-artigen Rezeptors (TLR) aufweist, zur Verwendung beim Behandeln oder Hemmen von Neutropenie bei einem Individuum, das diese(s) benötigt, wobei die therapeutisch wirksame Menge von Pirfenidon und des mindestens einen Agonisten eines toll-artigen Rezeptors (TLR) in separaten Formulierungen vorgesehen ist.

## Revendications

1. Quantité thérapeutiquement efficace de pirfénidone et d'un ou plusieurs agonistes des récepteurs de type Toll (TLR), pour utilisation dans le traitement ou l'inhibition d'une neutropénie chez un sujet qui en a besoin.

2. Quantité thérapeutiquement efficace de pirfénidone, pour utilisation dans le traitement ou l'inhibition d'une neutropénie chez un sujet qui en a besoin, où un ou plusieurs agonistes des récepteurs de type Toll (TLR) ont été administrés audit sujet.

3. Un ou plusieurs agonistes des récepteurs de type Toll (TLR), pour utilisation dans le traitement ou l'inhibition d'une neutropénie chez un sujet qui en a besoin, où une quantité thérapeutiquement efficace de pirfénidone a été administrée audit sujet.

4. Utilisation d'une quantité thérapeutiquement efficace de pirfénidone et d'un ou plusieurs agonistes des récepteurs de type Toll (TLR) dans la préparation d'un médicament destiné à traiter ou inhiber une neutropénie chez un sujet qui en a besoin.

5. Utilisation d'une quantité thérapeutiquement efficace de pirfénidone dans la préparation d'un médicament destiné à traiter ou inhiber une neutropénie chez un sujet qui en a besoin, dans laquelle un ou plusieurs agonistes des récepteurs de type Toll (TLR) ont été administrés audit sujet.

6. Utilisation d'un ou plusieurs agonistes des récepteurs de type Toll (TLR) dans la préparation d'un médicament destiné à traiter ou inhiber une neutropénie chez un sujet qui en a besoin, dans laquelle une quantité thérapeutiquement efficace de pirfénidone a été administrée audit sujet.

7. Pirfénidone ou un ou plusieurs agonistes de TLR pour utilisation selon la revendication 1, ou utilisation selon la revendication 4, où ladite pirfénidone et lesdits un ou plusieurs agonistes de TLR sont destinés à une administration simultanée.

8. Pirfénidone ou un ou plusieurs agonistes de TLR pour utilisation selon la revendication 1, ou utilisation selon la revendication 4, où ladite pirfénidone et lesdits un ou plusieurs agonistes de TLR sont destinés à une administration sous forme de formulations séparées.

9. Pirfénidone ou un ou plusieurs agonistes de TLR pour utilisation selon la revendication 1, ou utilisation selon la revendication 4, où ladite pirfénidone et lesdits un ou plusieurs agonistes TLR sont co-formulés.

10. Pirfénidone ou un ou plusieurs agonistes de TLR pour utilisation selon les revendication 1, 2 ou 3, ou utilisation selon les revendications 4, 5 ou 6, où le sujet est un humain.

11. Pirfénidone ou un ou plusieurs agonistes de TLR pour utilisation selon les revendications 1, 2, 3, 7, 8, 9 ou 10, ou utilisation selon les revendications 4, 5, 6 ou 10, où la pirfénidone ou un ou plusieurs agonistes de TLR, ou le médicament est destiné(e)/sont destinés à une administration dans une quantité efficace pour augmenter le nombre de neutrophiles chez le sujet.

12. Pirfénidone ou un ou plusieurs agonistes de TLR pour utilisation selon les revendications 1, 2, 3, 7, 8, 9 ou 11, ou utilisation selon les revendications 4, 5, 6 ou 11, où la quantité thérapeutiquement efficace est inférieure à 50 % d'une quantité qui provoque un effet secondaire indésirable chez le sujet.

13. Pirfénidone ou un ou plusieurs agonistes de TLR pour utilisation selon les revendications 1, 2 ou 3, ou utilisation selon les revendications 4, 5 ou 6, où la pirfénidone ou un ou plusieurs agonistes de TLR ou le médicament est destiné(e)/sont destinés à une administration orale.

14. Pirfénidone ou un ou plusieurs agonistes de TLR pour utilisation selon les revendications 1, 2 ou 3, ou utilisation selon les revendications 4, 5 ou 6, où la pirfénidone ou un ou plusieurs agonistes de TLR ou le médicament est destiné(e)/sont destinés à une administration deux fois par jour.

15. Pirfénidone ou un ou plusieurs agonistes de TLR pour utilisation selon les revendications 1, 2 ou 3, ou utilisation selon les revendications 4, 5 ou 6, où la pirfénidone ou un ou plusieurs agonistes de TLR ou le médicament est destiné(e)/sont destinés à une administration trois fois par jour.

16. Pirfénidone pour utilisation selon la revendication 1 ou 2, ou utilisation selon la revendication 4 ou 5, où la pirfénidone ou le médicament est destiné(e) à fournir la pirfénidone à une dose d'environ 100 à environ 400 milligrammes.

17. Pirfénidone pour utilisation selon la revendication 1 ou 2 ou utilisation selon la revendication 4 ou 5, où la pirfénidone ou le médicament est destiné(e) à administrer la pirfénidone de telle sorte que la prise quotidienne est d'environ 800 à environ 4 000 mg/jour.

18. Pirfénidone pour utilisation selon la revendication 1 ou 2, ou utilisation selon la revendication 4 ou 5, dans laquelle la pirfénidone ou le médicament est destiné(e) à administrer la pirfénidone de telle sorte que la prise quotidienne est d'environ 1 200 mg/jour ou plus.

19. Pirfénidone ou un ou plusieurs agonistes de TLR pour utilisation selon les revendications 1, 2 ou 3 ou utilisation selon les revendications 4, 5 ou 6, où ladite neutropénie est une neutropénie sévère.

20. Pirfénidone ou un ou plusieurs agonistes de TLR pour utilisation selon les revendications 1, 2 ou 3, ou utilisation selon les revendications 4, 5 ou 6, où ladite neutropénie est choisie dans le groupe constitué par la neutropénie associée à une chimiothérapie, la neutropénie associée à une thérapie d'oncologie classique, la neutropénie induite par les médicaments, la neutropénie induite par la maladie, la neutropénie génétique, la neutropénie induite par une toxine, la neutropénie congénitale, la neutropénie cyclique, la neutropénie idiopathique et la neutropénie induite par les rayonnements.

21. Pirfénidone ou un ou plusieurs agonistes de TLR pour utilisation selon les revendications 1, 2 ou 3, ou utilisation selon les revendications 4, 5 ou 6, où les un ou plusieurs agonistes de TLR comprennent au moins un agoniste de TLR7.

22. Pirfénidone ou un ou plusieurs agonistes de TLR pour utilisation, ou utilisation selon la revendication 21, où l'agoniste de TLR7 est choisi dans le groupe constitué par la 7-thia-8-oxoguanosine, la 7-dézaguanosine, la 7-allyl-8-oxoguanosine, la 7-dézaguanosine, l'imiquimod et R848.

23. Kit d'éléments comprenant :
une quantité thérapeutiquement efficace de pirfénidone ; et
un ou plusieurs agonistes des récepteurs de type Toll (TLR) ; pour utilisation dans le traitement ou l'inhibition d'une neutropénie chez un sujet qui en a besoin, dans lequel la quantité thérapeutiquement efficace de pirfénidone et le ou plusieurs agonistes des récepteurs de type Toll (TLR) sont fournis dans des formulations séparées.
